(19) Europäisches Patentamt
European Patent Office
Office européen des brevets

(11) **EP 3 191 830 B1**

(12) **EUROPEAN PATENT SPECIFICATION**

(45) Date of publication and mention of the grant of the patent:
**03.10.2018 Bulletin 2018/40**

(21) Application number: **15757467.4**

(22) Date of filing: **25.08.2015**

(51) Int Cl.:
*G01N 29/02* (2006.01)    *G01N 29/22* (2006.01)
*G01N 29/24* (2006.01)    *G01N 29/44* (2006.01)
*G01L 9/00* (2006.01)    *G01N 29/036* (2006.01)

(86) International application number:
**PCT/EP2015/069444**

(87) International publication number:
**WO 2016/037847 (17.03.2016 Gazette 2016/11)**

(54) **RESONANT MEMBRANE GAS SENSOR AND NON-TRANSITORY MACHINE-READABLE STORAGE MEDIUM THEREFORE**

RESONANTER MEMBRANGASSENSOR UND NICHTFLÜCHTIGES MASCHINENLESBARES SPEICHERMEDIUM DAFÜR

CAPTEUR RÉSONANT À MEMBRANE ET SUPPORT DE STOCKAGE LISIBLE PAR MACHINE NON TRANSITOIRE ASSOCIÉ

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **09.09.2014 US 201414480986**

(43) Date of publication of application:
**19.07.2017 Bulletin 2017/29**

(73) Proprietor: **ams International AG
8640 Rapperswil-Jona (CH)**

(72) Inventors:
• **VAN DER AVOORT, Casper
5583 TG Waalre (NL)**
• **BESLING, Willem Frederik Adrianus
5615 JN Eindhoven (NL)**

(74) Representative: **Epping - Hermann - Fischer Patentanwaltsgesellschaft mbH
Schloßschmidstraße 5
80639 München (DE)**

(56) References cited:
**EP-A2- 2 700 928      WO-A1-2009/071746
US-A1- 2002 115 198**

**Description**

The present application is related to a device comprising a gas sensor and a pressure sensor, and a tangible and non-transitory machine-readable storage medium encoded with instructions thereon for execution by a device for measuring carbon dioxide levels.

The patent application refers to resonant $CO_2$ sensing with mitigation of cross-sensitivities. Various exemplary embodiments disclosed herein relate generally to $CO_2$ sensing.

Electronic sensors may monitor air quality or gas composition in many ways; several physical properties of a gas mixture will change as the composition changes. For example, a sensor may be based on detecting the thermal conductivity of a gas mixture using a heated wire and measuring the heat dissipation of this wire to the surroundings. Specifically, $CO_2$ sensing may work in a number of ways. Two current methods include a material-based method where a material absorbs $CO_2$ and as a consequence changes electrical capacitance; and another where wire temperature is read by measuring electrical resistance - changes in the air mixture around a heated free-hanging wire result in changing the thermal conductivity of the medium, and hence the cooling of the heated wire. Another method is also known, resonant sensing of $CO_2$. Generally, this method requires a functional material on a MEMS element that resonates, detecting mass changes which result in frequency changes.

Document WO 2009/071746 A1 refers to a device for measuring pressure, variation in acoustic pressure, magnetic field, acceleration, vibration or composition of a gas. The gas sensor measures both the speed and damping of sound in a gas. A sensor comprises an ultrasound transmitter, a resonating sound cavity above the ultrasound transmitter and a reflector cavity above the resonating sound cavity. An ultrasound reflecting cover is on top of the reflector cavity and comprises breathing holes.

Document EP 2 700 928 A2 describes a pressure sensor having a membrane suspended over a cavity. The membrane has a plurality of openings such that gas can pass into and out of the cavity. The membrane has a resonance frequency. The membrane operates to flow gas viscously via the openings, with the viscosity of the gas and flow resistance of the openings used to detect a pressure characteristic of the gas in the chamber. Moreover, the viscosity and flow resistance are also used as an indication of the composition of the gas, using a known pressure.

Document US 2002/0115198 A1 describes a microfabricated ultrasound array for use as resonant sensors. A resonant micro mechanical membrane sensor comprises a silicon wafer substrate, a membrane and a sensor cavity. The interior of the cavity is vented to outside atmosphere by holes traversing the substrate. Electrostatic drive forces cause the membrane to resonate, and a binding event is detected.

An object of the present patent application is to provide a device comprising a gas sensor and a pressure sensor, and a tangible and non-transitory machine-readable storage medium encoded with instructions thereon for execution by a device for measuring carbon dioxide levels with high accuracy.

The object is solved by the subject-matter of the independent claims. Further embodiments are described in the dependent claims.

In an embodiment, a device may take advantage of the availability of accurate sensing of frequency by being sensitive to the average molar mass of a gas mixture, where the molar mass is reflected in the resonance frequency of the device. However, as for any gas sensor, the response will be varying with multiple influences, for example, gas pressure, temperature, and/or relative humidity. A molar mass sensor will also compensate for cross-sensitivities. Many methods require that the gas be in a particular state relative to the sensor, for example, a low vacuum or small space. Controlling the flow of gas in a detector may be difficult. At higher pressures and larger spaces (in the micro-meter range), molecules will collide with each other before hitting the sensor surface.

In ideal conditions, e.g. in the laboratory or other controlled conditions, there are several ways to measure the $CO_2$ content of a gas mixture. However, in the field, conditions are rarely ideal. For example, molar mass influences the average speed of particles (i.e., molecules in collision) in air. This in turn effects the collisions or impacts of particles on the walls (e.g. sensors) they encounter. The dynamic effects may be expressed as an airfilm stiffness, as well as an air film damping constant. The quality factor or Q-factor, the measurement of the resonant system's relative bandwidth, affects the range in which a system can detect gasses - in order to be able to measure small frequency shifts it is important to have sufficiently high Q.

For example, in devices operating in a vacuum, resonance frequency can be determined with high resolution (sub-ppm accuracy in detection of frequency shift) using a resonator with Q=40000. However, Q drops significantly when changing the ambient from vacuum to regular air. In air, that same resonator may exhibit a Q=3900. With this resonator, 1 ppm-level frequency detection is possible. However, a pressure sensor membrane uses a different type of resonator (bending mode rather than bulk mode), and reaches suitable Q levels only in vacuum using a sealed membrane. Requiring a vacuum or other specific environmental variables in order to reach suitable Q levels may be impractical for use in real-world conditions for a host of reasons, not least because it may be necessary to detect changing conditions in open air. An open structure has a resonance frequency that depends on gas pressure. However, the Q level at ambient pressure may be around 200 due to damping losses in air (due to air viscosity). Even with Q=200, frequency resolution allows detection of 20 ppm relative shift in resonance frequency. However, this means that in ambient conditions, a $CO_2$ change of 200 ppm particles results in a 12 ppm frequency change, which is already close to this detection limit of current devices. With oxygen and nitrogen as major components, the influence in the change of concentration of any of the other gases means that only small changes are to be expected in the molar mass. It is desired to be able to detect frequency shifts of the order of 10s of ppms, that is, when only the $CO_2$ concentration would have an effect on frequency. 10s of ppms is an abbreviation meaning that the frequency shifts are in the order of magnitude of tens such as 30, 40, 50 etc. of ppm.

In light of the present desire for frequency-based $CO_2$ sensing, a brief summary of various exemplary embodiments is presented. Some simplifications and omissions may be made in the following summary, which is intended to highlight and introduce some aspects of the various exemplary embodiments, but not to limit the scope of the invention. Detailed descriptions of a preferred exemplary embodiment adequate to allow those of ordinary skill in the art to make and use the inventive concepts will follow in later sections.

Various exemplary embodiments relate to the frequency shift of an oscillating membrane in relation to the amount of $CO_2$ molecules in the air around the membrane. The frequency shift may be proportional to temperature, pressure and humidity fluctuations in the air. In some embodiments, the resonant $CO_2$ sensor may function as an add-on to a combination of sensors for temperature, humidity and pressure. Various exemplary embodiments disclosed herein relate generally to a method to overcome cross-sensitivity to relative humidity.

Various exemplary embodiments relate to a device to measure carbon dioxide ($CO_2$) levels, including a first oscillator loop comprising a first sensor to measure air pressure, wherein the first sensor includes a first sealed membrane, wherein the first sealed membrane overlays a sealed first cavity; a second oscillator loop including a second sensor to measure the resonance frequency of a second unsealed oscillating membrane, wherein the second unsealed membrane overlays a second cavity in contact with the air outside of the second sensor; and a mixer accepting as input a first frequency measurement output from the first oscillator loop and a second frequency measurement output from the second oscillator loop, outputting the difference of the first frequency measurement and the second frequency measurement; and a circuit accepting as input the difference and outputting a carbon dioxide measurement.

In various alternate embodiments, the first membrane and the second membrane are identical in size, shape, and manufacturing process.

In various embodiments, the first membrane and the second membrane measure 200 x 200 $\mu m^2$.

The first membrane of the first sensor and the second membrane of the second sensor may be rectangular.

Alternatively, the first membrane and the second membrane have side lengths out of an interval between 50 $\mu m$ to 1000 $\mu m$. Alternatively, the first membrane and the second membrane have side lengths out of an interval between 100 $\mu m$ to 300 $\mu m$.

The side lengths of the different sides of the first membrane may be different. The side lengths of the different sides of the second membrane may be different.

The side lengths of the first membrane may be different or equal to the side lengths of the second membrane.

Alternatively, the first and/or the second membrane may be circular.

In alternate embodiments, the first sealed membrane is hermetically sealed and deflects under a pressure gradient.

In some alternate embodiments, deflecting under a pressure gradient includes deflecting under a pressure difference between inside the membrane and outside the membrane.

In some alternate embodiments, the second oscillator loop further includes a first top electrode and a second bottom electrode.

The second sensor may comprise the first top electrode and the second bottom electrode. Various exemplary embodiments relate to a device to measure carbon dioxide ($CO_2$) levels, including a first sensor to measure air pressure, wherein the first sensor includes a sealed membrane, wherein the sealed membrane overlays a sealed first cavity; a second sensor to measure the resonance frequency of an unsealed oscillating membrane, wherein the second sensor comprises a heater, wherein the unsealed membrane overlays a second cavity in contact with the air outside of the second sensor, and wherein the heater is in contact with the interior surface of the second cavity; and a mixer accepting as input a first frequency measurement output from the first sensor and a second frequency measurement output from the second sensor, and outputting the difference of the first frequency measurement and the second frequency measurement; and a circuit accepting as input the difference and outputting a carbon dioxide measurement.

In various alternate embodiments, the first membrane and the second membrane are identical in size, shape, and manufacturing process.

In various embodiments, the first membrane and the second membrane measure 200 x 200 $\mu m^2$.

In alternate embodiments, the first sealed membrane is hermetically sealed and deflects under a pressure gradient.

In some alternate embodiments, the second oscillator loop further includes a first top electrode and a second bottom electrode. The second sensor may comprise the first top electrode and the second bottom electrode.

Various exemplary embodiments relate to a tangible and non-transitory machine-readable storage medium encoded with instructions thereon for execution by a device for measuring carbon dioxide ($CO_2$) levels, wherein said tangible and non-transitory machine-readable storage medium comprises instructions for taking a first air pressure measurement using a first sensor, wherein the first sensor comprises a sealed membrane; instructions for taking a second resonance frequency of air measurement using a second sensor, wherein the second sensor comprises an unsealed oscillating membrane, wherein the unsealed membrane overlays a cavity in contact with the air outside of the second sensor; instructions for computing a difference of the first frequency measurement and the second frequency measurement; and instructions for computing a carbon dioxide measurement based on the difference.

The instructions may be performed on-line.

Alternative embodiments comprise instructions for, just before measuring the resonance frequency of air using the second sensor, heating the second cavity; and instructions for reading the temperature of the first membrane.

In some embodiments, instructions for taking a second resonance frequency of air measurement using a second sensor further comprise instructions for initiating electrostatic mechanical actuation of a top electrode of the device.

In other embodiments, electrostatic mechanical actuation of a top electrode further comprises applying a DC plus AC voltage over two electrodes of the device.

Various exemplary embodiments relate to a method of measuring carbon dioxide ($CO_2$) levels, the method including taking a first air pressure measurement using a first sensor, wherein the first sensor includes a sealed membrane; taking a second resonance frequency of air measurement using a second sensor, wherein the second sensor includes an unsealed oscillating membrane, wherein the unsealed membrane overlays a cavity in contact with the air outside of the second sensor; computing the difference of the first frequency measurement and the second frequency measurement; and computing a carbon dioxide measurement based on the difference.

Various exemplary methods further include, just before measuring the resonance frequency of air using the second sensor, heating the second cavity; and reading the temperature of the first membrane.

In various alternative embodiments, the step of taking a second resonance frequency of air measurement using a second sensor further includes electrostatic mechanical actuation of a top electrode.

The method is performed on-line and continuously. The steps were periodically repeated.

In various exemplary embodiments, electrostatic mechanical actuation of a top electrode further includes applying a DC plus AC voltage over two electrodes.

The first sensor may be realized as a pressure sensor. The second sensor may be realized as a gas sensor.

In an embodiment, a gas sensor comprises a cavity. The gas sensor is configured to sense at least one of a molar mass, a density and a viscosity of a gas mixture in the cavity.

Thus, the gas sensor may detect a physical or mechanical quantity or physical or mechanical property of the gas mixture in the cavity. Thus, the gas sensor is free of an absorbing layer that is designed to absorb the gas to be measured. Advantageously, a drift of the gas sensor, which is sometimes caused by changing properties of the absorbing layer, is avoided. The gas sensor uses a mechanical and/or physical principle, and not a chemical principle, for gas measurement. The molar mass, the viscosity and the density of the gas mixture that are determined by the gas sensor are a function of each of the gases comprised by the gas mixture in the cavity.

The gas sensor may be used for detecting at least one of the molar mass, the density and the viscosity of a gas mixture, for example for process monitoring or quality control such as the monitoring of industrial gas mixtures, natural gas, domestic gas, synthetic gas, compressed air and/or gas mixtures used in medical applications. By determining the molar mass of the gas mixture, the relative molecular mass (also called relative molar mass or molecular weight) may be determined also. The purity of the gas mixture may be determined.

The gas mixture may consist of exactly one gas having the molar mass. If the gas mixture consists of at least two gases, the average molar mass is determined by the gas sensor. However, "molar mass" is also used for "average molar mass" in the patent application.

In an embodiment, the concentration of most of the gases in the gas mixture is known or approximately known in a certain application. Only the concentration of one gas or of a small number of gases may be not known and may be variable in the gas mixture in said application. The gas sensor may be designed to determine the concentration of one gas based on the determination of the molar mass, the viscosity or the density of the gas mixture in the cavity. The gas may be carbon dioxide, humidity or another gas.

In an embodiment, the gas sensor comprises an oscillating membrane. The oscillating membrane is fabricated as an unsealed oscillating membrane. The unsealed oscillating membrane overlays the cavity. The cavity is in contact with a gas mixture outside of the gas sensor, such as the air outside of the gas sensor.

In an embodiment, a first surface of the unsealed oscillating membrane is in contact with the gas mixture outside of the gas sensor. The unsealed oscillating membrane comprises at least one gap, opening, hole or aperture. A second surface of the membrane and, thus, the cavity is in contact with the gas mixture outside of the gas sensor. The gas mixture in the cavity may be identical with the gas mixture outside of the gas sensor.

The unsealed oscillating membrane performs oscillations which depend on the molar mass and/or the density and/or the viscosity of the gas mixture in the cavity. The mechanical/physical properties of the gas mixture, such as molar mass, density and viscosity, have an influence on the oscillations of the unsealed oscillating membrane. They may have influence on at least one of an amplitude, a phase, a resonance frequency and a quality factor of the oscillations which are measured by the gas sensor.

In an embodiment, the gas sensor measures the resonance frequency of the unsealed oscillating membrane. The resonance frequency of the gas sensor is used to determine the molar mass and/or the viscosity and/or the density of the measured gas. Advantageously, a resonance frequency can be determined with high accuracy.

In an embodiment, the gas sensor uses the squeezed film effect of the gas mixture that is entrapped in the cavity. The cavity is realized as a shallow cavity. A height of the cavity is small. In an embodiment, the height is less than 5 $\mu$m. Alternatively, the height is less than 2 $\mu$m. Alternatively, the height is less than 0.5 $\mu$m. The small value of the height results in a squeezing of the gas mixture in the cavity. The cavity is configured such that the gas mixture in the cavity behaves like a squeezed film. The height may be named gap of the cavity.

In an embodiment, the gas sensor comprises a top electrode and a bottom electrode. The cavity is arranged between the top electrode and the bottom electrode. The gas sensor is configured to provide an electrostatic mechanical actuation of the top electrode. The top electrode is arranged in the unsealed oscillating membrane. An AC and/or a DC voltage is applied between the top electrode and the bottom electrode. When a non-zero voltage is applied between the top electrode and the bottom electrode, the top electrode is attracted towards the bottom electrode. The top electrode and the bottom electrode are not attracted to each other only in the case of a voltage having 0 V between the bottom electrode and the top electrode.

In an embodiment, a frequency of an alternating voltage or current applied between the top electrode and the bottom electrode is varied until the frequency of the voltage or current is equal to the resonance frequency of the unsealed oscillating membrane. In case of the resonance frequency, the amplitude of the oscillations of the unsealed oscillating membrane is higher than for frequencies which are lower or higher than the resonance frequency.

In an embodiment, the gas sensor comprises a heater. The heater is close to or in contact with the cavity. The heater may be comprised by the unsealed oscillating membrane. Alternatively, the heater may be on a bottom side of the cavity. The heater may be on a side of the cavity which is opposite to the unsealed oscillating membrane.

In an embodiment, the temperature is cycled using the heater. Thus, the temperature of the gas mixture in the cavity is cycled by the heater. The electric power provided to the heater is modulated to cycle the temperature. The pressure of the gas mixture in the volume of the cavity is approximately constant and is independent from the temperature. Thus, by cycling the temperature of the gas mixture in the cavity, the number of molecules or atoms of the gases in the gas mixture, and thus the concentration of each gas, is changed. The number of molecules or atoms of the gases in the gas mixture and the concentration of the different gases is proportional to the inverse $1/T$ of the absolute temperature. Thus, the molar mass and/or the density and/or the viscosity of the gas mixture is varied by the cycling of the temperature. A first value of the resonance frequency may be measured at a low temperature and a second value of the resonance frequency may be measured at a high temperature. More information about the gas mixture can be determined using the first and the second value in comparison to a measurement of the resonance frequency at a constant temperature.

In an embodiment, a device comprises the gas sensor and a pressure sensor. The pressure sensor is located in vicinity to the gas sensor.

The device may be designed to determine at least one of the molar mass, the density and the viscosity of the gas mixture. Alternatively or additionally, the device may be designed to determine the concentration of one gas using the information provided by the gas sensor and the information provided by pressure sensor. The molar mass, the density and the viscosity of the gas mixture not only depends on a relative concentration of the different gases in the gas mixture but also on the pressure inside the cavity. The pressure sensor provides a pressure sensor signal that is a function of the pressure in the gas mixture outside of the gas sensor. Since the gas sensor comprises the unsealed oscillating membrane, the pressure in the cavity is equal to the pressure outside of the gas sensor. Thus, the pressure sensor signal is a function of the pressure of the gas mixture in the cavity. The influence of the pressure on the determination of a gas concentration can be reduced by using the pressure sensor signal in addition to a gas sensor signal provided by the gas sensor, such as the resonance frequency of the unsealed oscillating membrane.

In an embodiment, a semiconductor body comprises the gas sensor. The gas sensor is fabricated on a surface of the semiconductor body. The semiconductor body may be made out of silicon. The gas sensor may be a silicon sensor.

The semiconductor body may also comprise the pressure sensor. The pressure sensor may be fabricated on the same surface of the semiconductor body on which the gas sensor is fabricated.

Alternatively, a further semiconductor body may comprise the pressure sensor. The further semiconductor body may be made out of silicon. The gas sensor and the pressure sensor may be fabricated on different semiconductor bodies.

It should be apparent that, in this manner, various exemplary embodiments enable $CO_2$ sensing.

In order to better understand various exemplary embodiments, reference is made to the accompanying drawings, wherein:

FIG. 1       provides a chart illustrating the distinction between viscous laminar flow and free molecular flow with regard to the expected path travelled by a molecule before striking another molecule;

FIG. 2A        illustrates an exemplary gas sensor open to the air;
FIG. 2B        illustrates an exemplary sealed air pressure sensor;
FIG. 2C        illustrates a further exemplary gas sensor;
FIG. 2D        illustrates a further exemplary pressure sensor;
FIG. 3         illustrates resonance frequency measurements for exemplary open and closed sensors;
FIG. 4         illustrates the relation between damping versus pressure for open and sealed devices; and
FIG. 5         illustrates an exemplary system or device for combining the measurements of two sensors.
Figures 6A and 6B    illustrate two exemplary tables showing the major components of air.
Figures 6C and 6D    illustrate exemplary tables showing the dependency of water content in air as a function of relative humidity and temperature.

In view of the foregoing, it would be desirable to implement a device to measure $CO_2$ content in air that does not rely on any specific pressure, temperature, or humidity. Only a change in molar mass of the gas mixture is detected. In particular, it would be desirable to create a method to separate the desired signal from other ambient parameters.

As for any gas sensor, measurements will vary due to multiple influences, for example, gas pressure, temperature, and relative humidity. These cross-sensitivities may be mitigated to achieve detection of $CO_2$ ppm level fluctuations in ambient air in the order of 200 ppm.

Referring now to the drawings, in which like numerals refer to like components or steps, there are disclosed broad aspects of various exemplary embodiments.

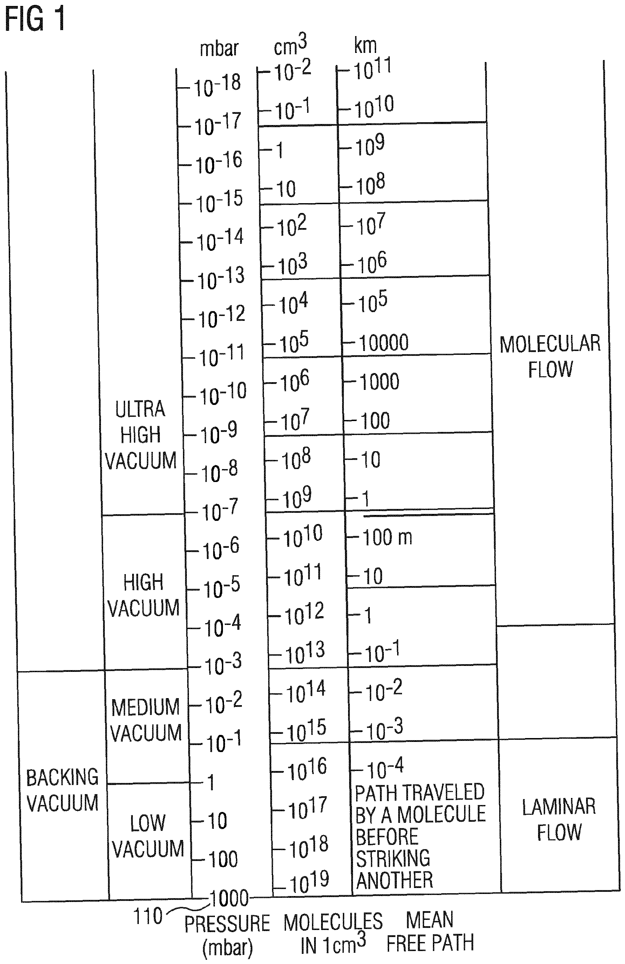

FIG. 1 illustrates the distinction between viscous laminar flow and free molecular flow with regard to the expected path travelled by a molecule before striking another molecule. When gas is in a state of free molecular flow, the mean free path of molecules is larger than the largest volume dimension of the encapsulation around the gas. One expected result is that molecules collide with the walls, not with each other. Such a state corresponds to low vacuum and/or small distance between the walls in which the gas is enclosed. At regular air pressures, gas acts in a state of viscous (laminar) flow.

At higher pressures (leftmost region of FIG. 1, 110) molecules will collide with each other before hitting a wall or other surface such as a sensor surface. On a crude scale, illustrated in FIG. 1, the free path length at the assumed standard sea level pressure of 1000 mbar is approximately 100 nm.

In the micro-meter range of free path length, such as that separating the walls in the micro structure of a detector, a molecular flow up to a level of above ~20 mbar would be expected. Note that in a gap of around 1 $\mu$m the molecules will not travel collision-free from one surface to another, but will experience only a handful of collisions. Therefore, for gaps of around 1 $\mu$m, a measurement of the mean velocities of molecules may be sufficiently accurate to be valuable. Although the gap height may be reduced to the mean free path length of molecules in ambient air conditions (i.e. ~100 nm), this may pose further constraints on manufacturing process control.

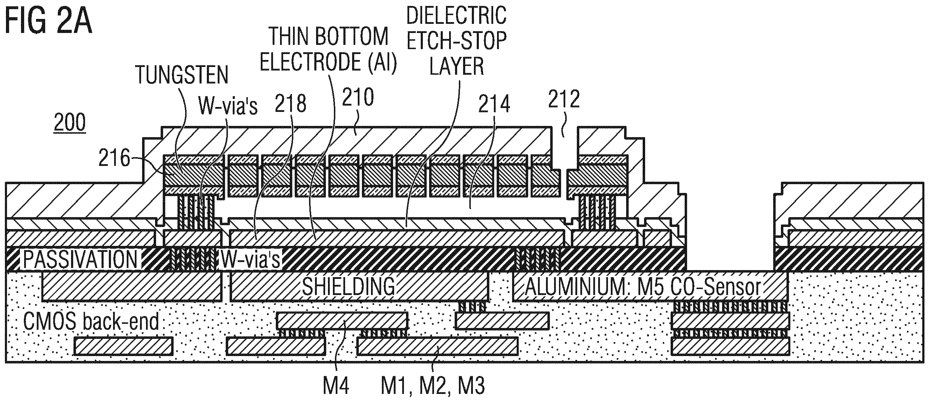

FIG. 2A illustrates an exemplary gas sensor 200 open to the air, in which the membrane 210 is in contact with the air through a gap 212 in the membrane 210, and thus is not deflected by an externally applied air pressure. The gas sensor 200 is also called second sensor.

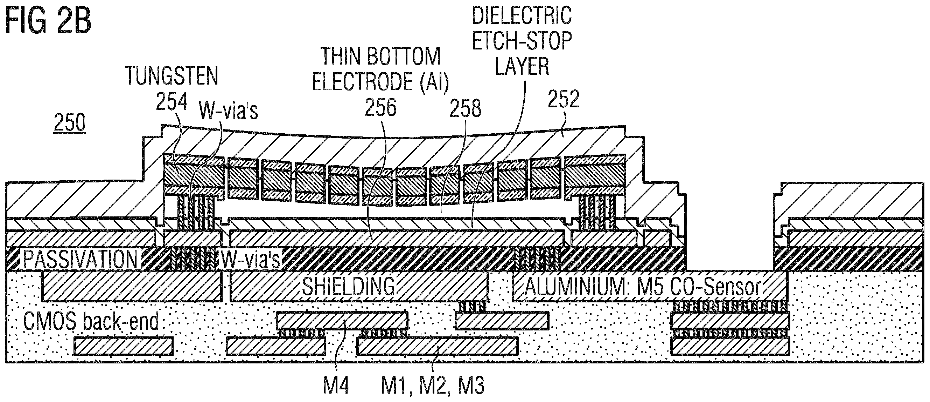

FIG. 2B illustrates an exemplary sealed air pressure sensor 250, where the membrane 252 is hermetically sealed and deflects under a pressure difference between inside the membrane (in the sealed cavity 258) and outside the membrane. The pressure sensor 200 is also called first sensor. In the exemplary sealed pressure sensor arrangement of FIG. 2B, the electrical capacitance of a capacitor comprising a top electrode 254, which may include tungsten (W), and a bottom electrode 256, which may include aluminium (Al), is a function of deflection of the top electrode 254, which in turn is a function of the pressure difference between inside and outside.

However, in order to detect ambient gases such as $CO_2$, the open structure illustrated in FIG. 2A is necessary so that ambient molecules can be detected after they pass through the gap 212 of the sensor structure, such that the micro cavity 214 is in contact with the ambient air. In such an arrangement, dynamic aspects of the thin film of air in the micro cavity 214 can be probed and measured by electrostatic mechanical actuation of the top electrode 216. Electrostatic mechanical actuation may be achieved by applying a DC plus AC voltage over the two electrodes 216, 218.

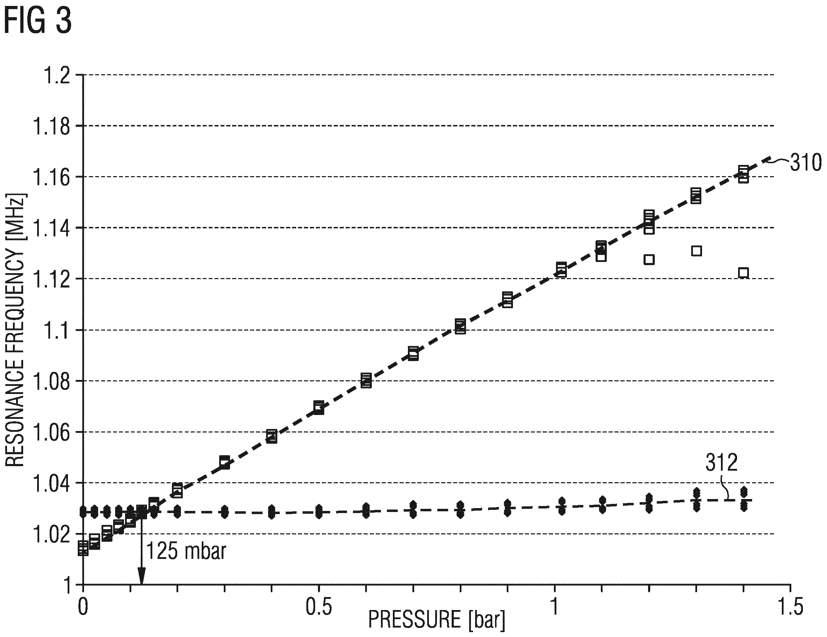

At the micro-meter range, the behavior of molecules at low pressures may be equal to that at higher pressure - transition from free molecular to viscous flow may not be visible. Thus, at a small scale it may not be necessary to take measurements under a vacuum in order to achieve proper measurements. FIG. 3 illustrates resonance frequency measurements for exemplary 200 x 200 $\mu$m$^2$ open and closed sensors 210, 250, as illustrated in FIGs. 2A and 2B where a micro cavity 214, 258 measures 0.6 $\mu$m high. Data for the open structure of FIG. 2A along line 310 illustrates measurement of the resonance frequency of the top electrode 216 or the membrane 210 as a function of the air pressure. Because the membrane 210 of the gas sensor 200 is open at gap 212, the pressure inside the cavity 214 is the same as the ambient pressure outside the gas sensor 200, which is shown in FIG. 3 at varied levels in a range from 1 to over 1400 mbar. The membrane 210 is realized as an unsealed oscillating membrane.

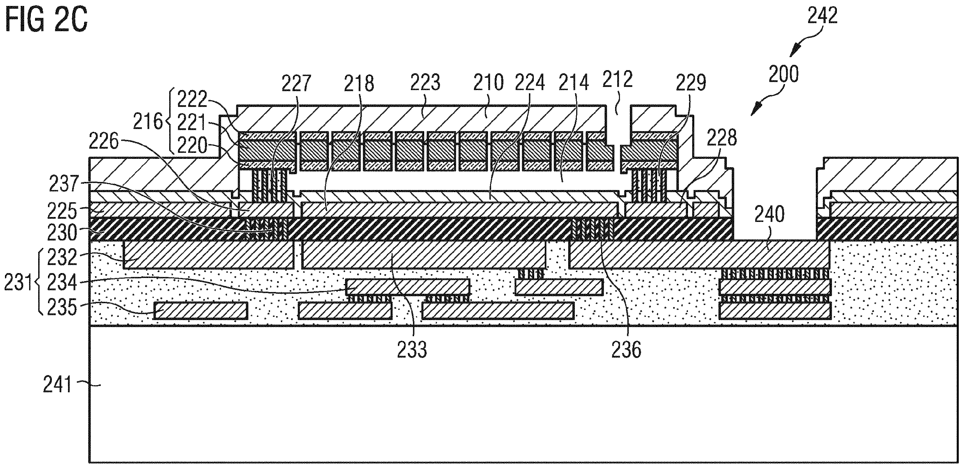

Figure 2C shows an exemplary embodiment of the gas sensor 200 which is a further development of the gas sensor shown in Figure 2A. The gas sensor 200 comprises the cavity 214 which can also be named "micro cavity" and the unsealed oscillating membrane 210. The unsealed oscillating membrane 210 comprises the top electrode 216. The gas sensor 200 also comprises the bottom electrode 218. The cavity 214 is enclosed by the unsealed oscillating membrane 210 and the bottom electrode 218. The unsealed oscillating membrane 210 comprises the gap 212 which can be also named "port", "aperture", "hole" or "opening". Alternatively, the unsealed oscillating membrane 210 comprises more than one gap/port/aperture/hole/opening. The gas mixture can flow from the outside of the gas sensor 200 to the cavity 214 through the at least one gap 212.

The top electrode 216 may be realized using three metallization films 220, 221, 222. A first film 220 is adjacent to the cavity 214. The first film 220 may be an adhesion layer. A second film 221 of the top electrode 216 may be implemented as a main metallization layer, for example out of tungsten or aluminium. A third film 222 of the top electrode 216 may be realized as an anti-reflective coating. The unsealed oscillating membrane 210 comprises a membrane isolating layer 223 that covers the top electrode 216. The gap 212 is realized in the top electrode 216 and in the membrane isolating layer 223.

The gas sensor 200 comprises a bottom isolating layer 224 that is arranged between the bottom electrode 218 and the cavity 214. The bottom isolating layer 224 isolates the bottom electrode 218 from the top electrode 216. The bottom isolating layer 222 is designed as a dielectric etch-stop layer. During the etching of a sacrificial layer for a fabrication of the cavity 214, the etchant stops at the bottom isolating layer 224. The bottom electrode 218 may be fabricated from a conductive layer 225, for example out of aluminium.

The gas sensor 200 comprises a first conducting line 226 that may be fabricated using the conductive layer 225. The first conducting line 226 is coupled to the top electrode 216 by a first via 227. Additionally, the gas sensor 200 comprises a second conducting line 228 fabricated from the conductive layer 225. The second conductive line 228 is coupled to the top electrode 216 by a second via 229.

Additionally, the gas sensor 200 comprises a passivation layer 230 on which the bottom electrode 218 is arranged. The first and the second conducting lines 226, 228 are also arranged on the passivation layer 230. Additionally, the gas sensor 200 comprises a metallization stack 231 which also can be called CMOS back-end. The metallization stack 231 comprises at least one metallization layer 232. The gas sensor 200 comprises a shielding structure 233 which may be fabricated using the first metallization layer 232. The shielding structure 233 is realized below the bottom electrode 218. The shielding structure 223 is separated from the bottom electrode 218 by the passivation layer 225.

The bottom electrode 218 is coupled by a via 236 to a conducting line comprised by the first metallization layer 232. Correspondingly, the first conducting line 226 is coupled by a via 237 to a conducting line comprised by the first metallization layer 232. The metallization stack 231 may comprise at least a second and a third metallization layer 234, 235. The first metallization layer 232 is coupled to the second and the third metallization layer 234, 235 by additional vias.

The gas sensor 200 may also comprise a carbon monoxide sensor 240. The carbon monoxide sensor 240 is realized inside the first metallization layer 232. The membrane isolating layer 223, the bottom isolating layer 224 and the passivation layer 225 comprise an opening such that gas can flow or diffuse to the carbon monoxide sensor 240. Alternatively, the gas sensor 200 is free of the carbon monoxide sensor 240.

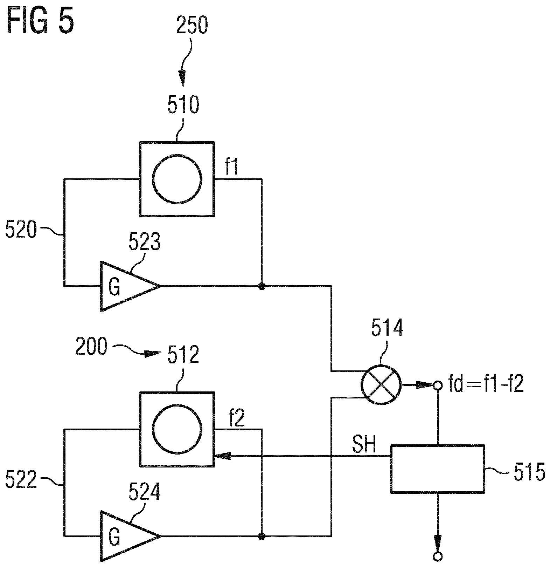

Moreover, the gas sensor 200 comprises a semiconductor substrate 241. The metallization stack 231 is arranged on the semiconductor substrate 241. The semiconductor substrate 241 may be made out of silicon. The semiconductor substrate 241 and the metallization stack 231 may comprise a circuitry, shown in detail in FIG. 5. The circuitry may be a CMOS circuit or CMOS circuits. The circuitry is coupled to the top and the bottom electrode 216, 218 of the gas sensor 200.

A semiconductor body 242 comprises the gas sensor 200. The gas sensor 200 is realized as a silicon sensor. The gas sensor 200 is fabricated based on micro-electro-mechanical-system technology. Thus, the gas sensor 200 is a micro-electro-mechanical sensor, abbreviated as MEMS sensor. The cavity 214 is realized by etching of a sacrificial layer. Thus, the gas sensor 200 is fabricated using a surface micromachining technique. The height of the cavity 214 is determined by the thickness of the sacrificial layer. The height of the cavity 214 may be less than 5 $\mu$m. Alternatively, the height of the cavity 214 may be less than 2 $\mu$m or 0.5 $\mu$m. Due to the low height of the cavity 214, the gas mixture inside the cavity 214 behaves like a squeezed film, when the top electrode 216 moves towards the bottom electrode 218.

The unsealed oscillating membrane 210 performs mechanical oscillations which are mechanical vibrations. The movement of the middle of the membrane 210 is perpendicular to the surfaces of the membrane 210. The vibrations of the unsealed oscillating membrane 210 is caused by providing an electrical signal such as an alternating voltage or current to the top electrode 216 of the gas sensor 200. The unsealed oscillating membrane 210 may generate a longitudinal wave in the gas mixture of the cavity 214 such as an acoustic wave.

The gas sensor 200 may additionally comprise a temperature sensor, not shown, measuring the temperature near the cavity 214.

The gas sensor 200 may comprises a heater. The heater may be implemented as a resistive heater. The heater may be realized as a metal resistor. The heater may be part of the unsealed oscillating membrane 210. The heater may be realized by the top electrode 216. The top electrode 216 is contacted twice, namely by the first conducting line 226 and the first via 227 as well as by the second conducting line 228 and the second via 229. Thus, a current can be provided to the top electrode 216 for heating by the first and the second conducting line 226, 228. The current may be generated by the circuitry realized by the semiconductor substrate 214 and the metallization stack 231. Advantageously, the heater is thermally isolated by the cavity from the metallization stack 231 and the semiconductor substrate 240. Thus, only a small amount of power is required for heating of the gas mixture in the cavity 222.

In an alternative embodiment, not shown, the heater is fabricated on top of the membrane isolating layer 223. The heater is coupled by additional vias and structures in the conductive layer 225 to the circuitry.

Alternatively, the heater may be realized below the bottom electrode 218 or may use the bottom electrode 218 as a heater.

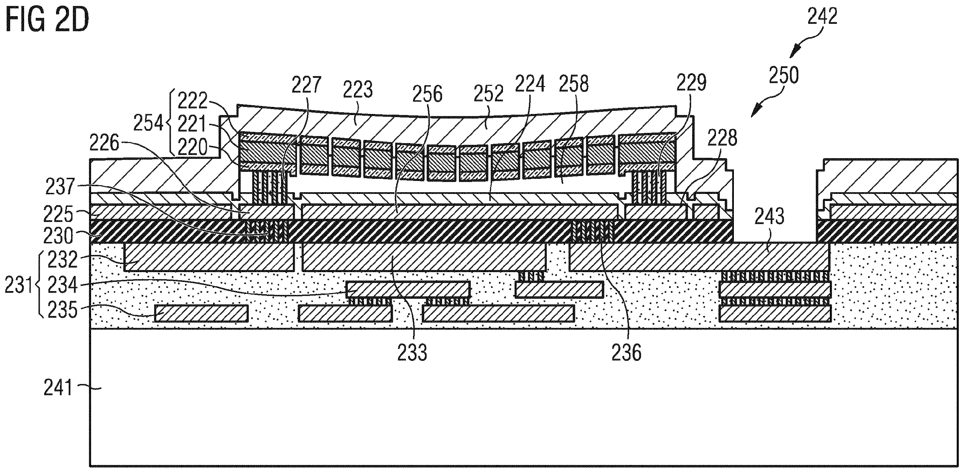

Figure 2D shows an exemplary embodiment of the pressure sensor 250 which is a further development of the embodiment shown in Figure 2B. The pressure sensor 250 is fabricated on the semiconductor substrate 241 on which also the gas sensor 200 is realized. Thus, one semiconductor body 242 comprises the gas sensor 200 as well as the pressure sensor 250. Thus, the layers of the pressure sensor 250 correspond to the layers of the gas sensor 200. The pressure sensor 250 comprises the sealed membrane 252. The sealed membrane 252 comprises the top electrode 254 of the pressure sensor 250. As explained in Figure 2C, the top electrode 254 comprises the first, the second and the third metallization film 220 to 222. Moreover, the sealed membrane 252 comprises the membrane isolating layer 223. The sealed membrane 252 is free of a gap, opening, hole or aperture. Thus, the gas mixture outside of the pressure sensor 250 is not able to diffuse or flow into the cavity 258 of the pressure sensor 250. The sealed membrane 252 is deflected by a pressure difference between a pressure of the gas mixture outside of the pressure sensor 250 and the gas mixture or vacuum in the cavity 258 of the pressure sensor 250. The top electrode 254 is coupled to a first conducting line 226 of the pressure sensor 250 by a via 227.

Additionally, the pressure sensor 250 comprises the bottom electrode 256. Moreover, the pressure sensor 250 comprises the bottom isolating layer 224. The bottom electrode 256 of the pressure sensor 250 is coupled to a conducting line of the first metallization layer 232 by a via 236. The bottom electrode 256 and the first conducting line 226 can be realized by the conductive layer 225. The pressure sensor 250 is fabricated using the same semiconductor substrate 241 as the gas sensor 200. The first conducting line 226 is coupled by a via 237 to a conducting line of the metallization layer 232. The metallization stack 231 is arranged between the semiconductor substrate 241 and the passivation layer 230.

The pressure sensor 250 comprises a shielding structure 233 which may be fabricated using the first metallization layer 232. The shielding structure 233 is realized below the bottom electrode 256. The shielding structure 223 is separated from the bottom electrode 256 by the passivation layer 225.

The pressure sensor 250 may also comprise a further carbon monoxide sensor 243.

A pressure sensor signal generated by the pressure sensor 250 is combined with a gas sensor signal provided by the gas sensor 200, for example, the resonance frequency of the gas sensor 200. The pressure sensor is realized as a capacitive pressure sensor. The capacitance value of the pressure sensor 250 depends on the pressure difference between the gas mixture outside of the pressure sensor 250 and the gas mixture or vacuum in the cavity 258. Thus, a circuit such as a capacitance-to-voltage converter can be used to generate the pressure sensor signal which may be a digital or an analog signal.

Alternatively, an oscillating circuit such as is shown in Figure 5 may be used to generate the pressure sensor signal. Thus, an alternating signal such as a current or voltage is provided to the top electrode 254 of the pressure sensor 250. A resonance frequency of the sealed membrane 252 is detected as the pressure sensor signal. Thus, a circuitry for operating the gas sensor 200 may be similar or equal to a circuitry operating the pressure sensor 250.

In an alternative, not-shown embodiment, a further semiconductor substrate is used for the fabrication of the pressure sensor 250. Thus, the pressure sensor 250 and the gas sensor 200 may be fabricated using different MEMS technologies. The pressure sensor 250 and the gas sensor 200 are arranged in vicinity on a common substrate such as a printed circuit board, a ceramic or a housing.

As can be seen in FIG. 3, the resonance frequency of the membrane interacting with the air cavity below it measured by open gas sensor 200 depends on gas pressure, such that

$$\omega^2 = \frac{k_{eff} + k_{film}}{m_{eff}} \quad and \; k\_film = \frac{c \, A \, p}{d} \quad ;$$

where $k_{film}$ equals air film stiffness, $k_{eff}$ equals mechanical stiffness, $m_{eff}$ equals the effective mass; and k_film includes a gas-type-dependent constant c, the frontal area A, pressure p and electrode separation d. The electrode separation d is equal to the height of the cavity 214. The square-root nature in frequency is not visible in the pressure range of FIG. 3. The slope of the frequency-vs-pressure line is proportional to the molar mass of the gas mixture. Therefore, the measurements of gas sensor 2006 may be assumed to be at nominal regular air pressure of approximately 1000 mbar, and then combined with the measurement of the real pressure - when the average pressure is known, the absolute resonance frequency f2 of the gas sensor 200 may be used to determine the molar mass of the measured gas.

The next calculation indicates what the change in frequency to be detected should be, in order to see a change of 200 ppm $CO_2$. Gas film stiffness and gas film damping coefficient may be determined as a function of pressure for different gases, for example, helium (M = 4), nitrogen (M = 28) and hexafluoroethane (M = 138). Squeeze film damping is also a function of the gas-type, but air film stiffness and hence the $k_{film}$-vs-p ratio scales with M. In fact, it scales as VM, meaning relative slopes of 2, 5.3, and 12 for a plot of the $k_{film}$-vs-p ratios of helium, nitrogen, and hexafluoroethane when taken at very low pressures, for example, between 0 and 1 mbar.

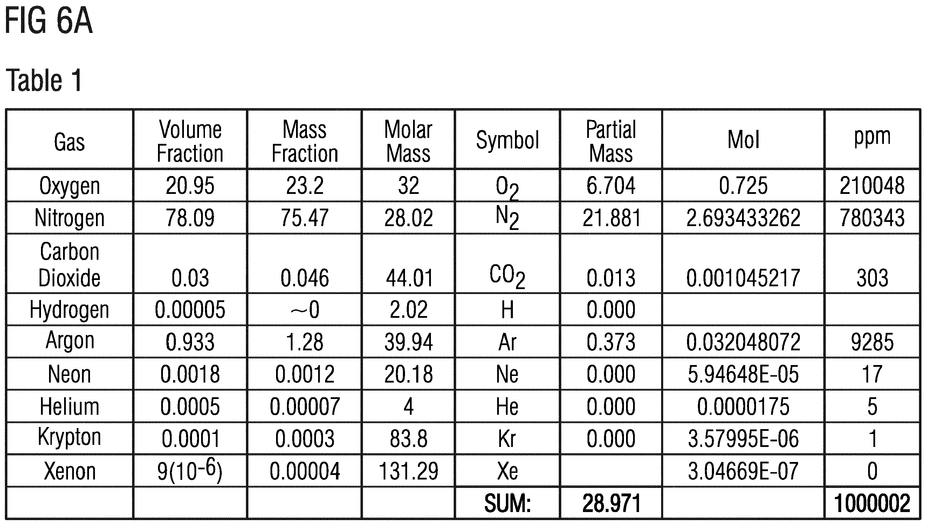

The major components of regular air are oxygen, nitrogen, carbon dioxide, hydrogen, argon, neon, helium, krypton, and xenon (water vapor, especially in the form of humidity, is also a component of ambient air, but is discussed later). The following table (Table 1 in FIG. 6A) illustrates a change in average molar mass for the major components, summing to an indicated overall molar mass (28.971), and their molar distribution; due to rounding, one million plus 2 parts per million are listed (note that $CO_2$ is assumed to have 303 ppm in this mixture).

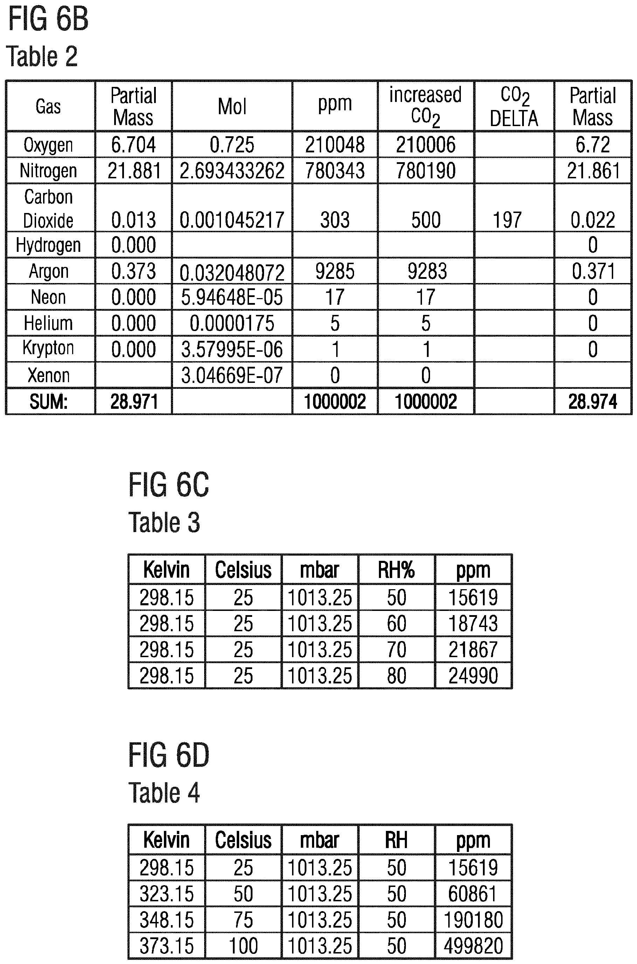

With oxygen and nitrogen as major components, the influence in the change of concentration of any of the other gases means that only small changes are to be expected in the molar mass. For example, where $CO_2$ is 303 ppm as demonstrated in Table 1, molar mass M = 28.971. Table 2 in FIG. 6B illustrates a change in the $CO_2$-level, while keeping the total amount of particles constant.

As is shown, changing the $CO_2$ concentration from 303 ppm to 500 ppm, a difference of 197 ppm, while assuming proportional accommodation by all other gases, results in a new molar mass. This is a worst-case estimate of the to-be-detected mass change, because actually it is more likely that $O_2$ will be traded for $CO_2$ by breathing, and $N_2$ will remain unaffected, resulting in a slightly more pronounced change in molar mass. In the exemplary situation shown in Table 2, the relative change is 0.011 % or 110 ppm, and the new molar mass M=28.974. Although this is a minute difference, a measurement made before and after the change at exactly the same pressure level would result in a noticeable change in frequency.

A change in molar mass reflects a change in the spring constant of the air film as reflected by the equation:

$$\omega^2 = \frac{k_{eff} + k_{film}}{m_{eff}}$$

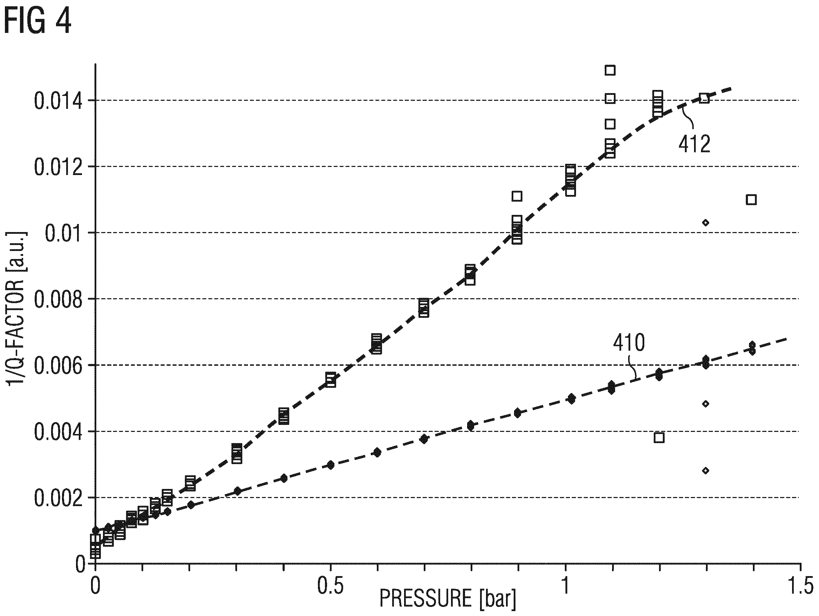

This value may be derived from a reading by the gas sensor 200 of frequency f, where f = √ω. For example, for a 240 micron square membrane 210 shown in the configuration of open sensor 200 in Fig. 2A, keff = 1.3 * 104 [N/m]. At 1 bar the airfilm has $k_{film}$ = 3.6 * 103 [N/m]. With effective mass $m_{eff}$ = 5.5 * 10-10 [kg], the resonance frequency for this exemplary device is $f_{res}$ = omega/2pi = 874 kHz. If the molar mass of the gas mixture were to change, the ratio of $k_{film}$-vs-p will change and hence the airfilm stiffness at one bar will be different. (See, e.g., Matthijs Suijlen, "Model-based design of MEMS resonant pressure sensors", NXP 2011, Thesis, http://alexandria.tue.nl/extra2/716458.pdf, last accessed August 14, 2014; esp. Fig. 4 (experiments where gas film stiffness and gas film damping coefficient are determined as a function of pressure, for different gases, with measurements only up to 1 mbar, residing in the regime of free molecular flow)). The proportional change on $k_{film}$ is 110 ppm, so the frequency with the above example will change from 874.000 kHz to 874.010 kHz, a change of 12 ppm.

FIG. 4 illustrates the relation between damping versus pressure for open and sealed devices such as sensors 200 and 250. As noted above, the Q factor affects the range in which a system or device can detect gasses - in order to be able to measure small frequency shifts it is important to have sufficiently high Q, but a pressure sensor membrane reaches suitable Q levels only in vacuum using a sealed membrane. As shown in FIG. 4, for pressure sensors with a sealed membrane, the Q factor (shown as damping factor 1/Q) is impacted by the outside pressure, as shown by line 410, but the frequency is nearly unchanged as a function of pressure as shown by line 312 in FIG. 3. Hence, there is no additional spring constant for sealed devices such as pressure sensor 250. However, open devices such as gas sensor 200 have a significantly higher frequency and a lower Q of ~200 due to the squeezed film effect of air that is present/entrapped in the cavity 214 (as shown by line 412). As shown in FIG. 4, for both open and closed devices 1/Q is a linear function of pressure. The difference between the slope of the 1/Q curves 410, 412, is proportional to the additional damping effect of the air molecules that are present in the cavity.

Air pressure, temperature, and humidity all will change the detectable resonance frequency. In order to detect frequency shifts on the order of 10 ppms when only $CO_2$ concentration would have an effect on frequency, the effects of these factors should be mitigated. A sensor such as exemplary gas sensor 200 may be modified to mitigate these factors and achieve conditions in which it is possible to measure smaller frequency shifts than would be possible when measuring unaltered outside air.

For example, if the membranes 210, 252 incorporated into gas sensor 200 and pressure sensor 250 may be well matched, e.g. identical except for the difference of being sealed or unsealed, in order that the measurements from them would be under near-identical external conditions. In such an arrangement, the pressure effect on the resonance frequency of the open membrane 210 may be compensated for with the sealed membrane 252 acting as an absolute pressure sensor 250 relative to the open sensor 200.

Also, note that temperature effects will have the same delta on membranes 210 and 252 - the difference signal will be much less depending on temperature (T). The delta in residual frequency $f_{res}$ between open and sealed membranes 210, 252 is a good measure to determine the changes in $f_{res}$ of the open membrane 210 because the effect of temperature variations will become small. As the resonance frequency of the sealed membrane 252 is not a function of pressure due to the fact that the cavity pressure is very low (as shown in FIG. 3) the actual slope of $f_{res}$ (P) is directly proportional to the molar density of the gas that is present inside the cavity 214. In order to detect a molar density change due to a gas composition variation of 200 ppm the error in the pressure measurement should be much smaller than 200 ppm. Currently known capacitive pressure sensors can achieve a relative accuracy of 2 Pa on 1 bar, or 20 ppm. The information on the Q-factor can also be used to extract information on damping which is dependent upon pressure and molecule mass.

Additionally, a method of mitigating moisture and humidity that may affect $CO_2$ detection is discussed below with relation to humidity effects and mitigation.

Because cross-sensitivities such as air pressure, temperature, and humidity determine the accuracy of the sensor, a system or device allowing the comparison of the resonance frequency of ambient gas and the degree of pressure to a significant degree of accuracy requires measurements from both an open-structure sensor 200 and a sealed-membrane sensor 250, taken simultaneously.

To discriminate the effect of gas-mixture-change from changes in pressure or temperature, a difference in frequencies may be detected as illustrated in FIG. 5, where a system or device combines the measurements of the sensors such as, for example, sensors 200 and 250. Two sensor membranes 510 and 512 are incorporated in two separate oscillator loops 520, 522, also called two oscillator groups. The resulting frequencies f1 (of the pressure sensor 250) and f2 (of the gas or $CO_2$ sensor 200) are fed to a mixer 514, yielding the difference frequency fd. As illustrated in Tables 1 and 2 and FIGs. 3 and 4, the difference frequency fd at constant pressure is proportional to change in $CO_2$. At changing pressure levels, detected by f1, a stored look-up table may be generated by an initial calibration procedure to set a reference point for the value of f2 at a defined $CO_2$ level.

As noted above, ambient air contains water molecules. Water vapor accounts for a significant number of molecules in the air. The molar mass of water is 18, compared to 44 for $CO_2$. The levels of water vapor tend to be very large compared to the few hundred ppm $CO_2$ that might be necessary to detect in many applications, e.g. in a $CO_2$ detector meant to warn humans of rising $CO_2$ levels before they become toxic. For example, Table 3 in FIG. 6C lists the ppm of water with varying relative humidity at room temperature and ~1 bar pressure:

The water content is also a strong function of temperature. For example, in Table 4 shown in FIG 6D, only temperature is varied:

While the ppm-versus-RH% is nearly linear, the curve of ppm-versus-temperature shows an exponential nature. At very high levels of RH% the water content expressed in ppm approaches 30000 for the condition of room temperature and 1 bar air pressure. If the gas mixture in cavity 214 includes an applied temperature well, the ppm-level of water in air may be modulated by modulating the temperature of the gas sensor 200. A heater such as a joule heater may be incorporated in the structure of gas sensor 200 close to or in contact with cavity 214, in order to evaporate all moisture that is adsorbed onto the membrane 210 just before measuring the resonance frequency.

As discussed above, the gas sensor 200 senses the molar mass of a gas mixture (i.e. air) in a shallow cavity 214. The molar mass is reflected in the resonance frequency. The frequency also depends on temperature, RH% and pressure, so separate sensors are used to measure these parameters. To remove the combined cross-sensitivity of water and temperature, the system or device may use a modulation method where the temperature, and hence the water content, is cycled using a heater. Note that the absolute water concentration in the air is not directly modulated by the increasing temperature if there is no direct (e.g., liquid) water source - however, if the water film is evaporated from the surface inside the cavity the concentration of the water vapor molecules will increase. The frequency of the sensor will follow the temperature modulation because of its own temperature dependency, in addition to an even larger magnitude of the induced change in molar mass because of the fluctuating water levels. However, these effects can be compensated for by using the difference frequency of the sealed and open membranes in sensors 200, 250. The temperature modulation will cause the absolute humidity level to be changed while the $CO_2$ ppm-level remains constant.

As illustrated in Figure 5, the system or device comprises the gas sensor 200 and the pressure sensor 250. The gas sensor 200 can also be named "second sensor", the pressure sensor 250 can also be named "first sensor". The system or device comprises a first and a second oscillator loop 520, 522. The first oscillator loop 520 comprises the pressure sensor 250 and the second oscillator loop 522 comprises the gas sensor 200. Additionally, the first oscillator loop 520 comprises a first amplifier 523. The second oscillator loop 522 comprises a second amplifier 524. The first and the second amplifier 523, 524 are realized as gain amplifiers.

An output of the first amplifier 523 is connected to a terminal of the pressure sensor 250. Another terminal of the pressure sensor 250 is connected to an input of the first amplifier 523. The terminal and the other terminal of the pressure sensor 250 may be the top and/or the bottom electrode 254, 256 of the pressure sensor 250. An output of the first amplifier 523 is coupled to a first input of the mixer 514.

An output of the second amplifier 524 is connected to a terminal of the gas sensor 200. Another terminal of the gas sensor 200 is connected to an input of the second amplifier 524. The terminal and the other terminal of the gas sensor 200 may be the top and/or the bottom electrode 216, 218 of the gas sensor 200. An output of the second amplifier 524 is coupled to a second input of the mixer 514.

A circuit 515 may be coupled to the output of the mixer 514 and calculates the gas concentration. The circuit 515 may comprise the look-up table. The circuit 515 may control the heater of the gas sensor 200 by a heater signal SH. The first and the second amplifier 523, 524, the mixer 514, the circuit 515, the gas sensor 200 and the pressure sensor 250 may be comprised by the semiconductor body 242. The system or device may detect for example carbon dioxide. If the concentration of carbon dioxide is constant or is known, the system or device may detect humidity, since water molecules are also a gas, or another gas. The gas sensor 200 and, thus, the system or device is actually sensitive to a change in molar mass of the gas mixture to which it is exposed.

According to the foregoing, various exemplary embodiments provide for compensating for cross-sensitivities related to a gas sensor. In particular, by implementing a multiple-sensor package, including a heater, and accounting for known effects.

It should be apparent from the foregoing description that various exemplary embodiments of the invention may be implemented in hardware and/or firmware. Furthermore, various exemplary embodiments may be implemented as instructions stored on a machine-readable storage medium, which may be read and executed by at least one processor to perform the operations described in detail herein. The circuit 515 may comprise the at least one processor. A machine-readable storage medium may include any mechanism for storing information in a form readable by a machine, such as a personal or laptop computer, a server, or other computing device. Thus, a machine-readable storage medium may include read-only memory (ROM), random-access memory (RAM), magnetic disk storage media, optical storage media, flash-memory devices, and similar storage media. The instructions and the mechanism are performed on-line.

It should be appreciated by those skilled in the art that any block diagrams herein represent conceptual views of illustrative circuitry embodying the principals of the invention. Similarly, it will be appreciated that any flow charts, flow diagrams, state transition diagrams, pseudo code, and the like represent various processes which may be substantially represented in machine readable media and so executed by a computer or processor, whether or not such computer or processor is explicitly shown.

The pressure sensor 250 is equal to the first sensor 250 continuously in the patent application. Additionally, the gas sensor 200 is equal to the second sensor 200 continuously in the patent application.

Although the various exemplary embodiments have been described in detail with particular reference to certain exemplary aspects thereof, it should be understood that the invention is capable of other embodiments and its details are capable of modifications in various obvious respects. As is readily apparent to those skilled in the art, variations and modifications can be affected while remaining within the scope of the invention. Accordingly, the foregoing disclosure, description, and figures are for illustrative purposes only and do not in any way limit the invention, which is defined only by the claims.

## Claims

1. A device, comprising
   a first oscillator loop (520) that comprises a pressure sensor (250),
   a second oscillator loop (522) that comprises a gas sensor (200),
   a mixer (514) accepting as input a first frequency measurement output (f1) from the first oscillator loop (520) and a second frequency measurement output (f2) from the second oscillator loop (552), outputting the difference of the first frequency measurement and the second frequency measurement, and
   a circuit (515) that is coupled to an output of the mixer (514),
   wherein the pressure sensor (250) comprises a sealed cavity (258) and a sealed membrane (252) that is deflected by a pressure difference between a gas mixture outside of the pressure sensor (250) and a gas mixture or vacuum in the sealed cavity (258) of the pressure sensor (250),
   wherein the gas sensor (200) comprises a cavity (214) and an unsealed oscillating membrane (210) having a resonance frequency (f2) and overlaying the cavity (214) in contact with the gas mixture outside of the gas sensor (200),
   wherein the gas mixture can flow from the outside of the gas sensor (200) to the cavity (214) through at least one opening (212) of the unsealed oscillating membrane (210), and
   wherein the device is configured to sense at least one of a molar mass, a density and a viscosity of the gas mixture in the cavity (214) overlaid by the unsealed oscillating membrane (210).

**2.** The device of claim 1,
wherein the cavity (214) is realized as a shallow cavity and due to the low height of the cavity (214), the gas mixture inside the cavity (214) behaves like a squeezed film, when a top electrode (216) of the gas sensor (200) moves towards a bottom electrode (218) of the gas sensor (200) such that the gas sensor (200) uses the squeezed film effect of the gas mixture that is entrapped in the cavity (214).

**3.** The device of claim 1 or 2,
wherein the gas sensor (200) comprises a top electrode (216) and a bottom electrode (218) and is configured for an electrostatic mechanical actuation of the top electrode (218).

**4.** The device of one of claims 1 to 3,
wherein the gas sensor (200) comprises a heater close to or in contact with the cavity (214).

**5.** The device of claim 4,
wherein the temperature is cycled using the heater.

**6.** A tangible and non-transitory machine-readable storage medium encoded with instructions thereon for execution by a device for measuring carbon dioxide ($CO_2$) levels, wherein said tangible and non-transitory machine-readable storage medium comprises:

- instructions for taking a first resonance frequency (f1) of air pressure measurement using a first oscillator loop (520) comprising a first sensor (250), wherein the first sensor (250) comprises a sealed membrane (252) and the sealed membrane (252) overlays a sealed first cavity (258) comprising a gas mixture or vacuum;
- instructions for taking a second resonance frequency (f2) of air measurement using a second oscillator loop (552) comprising a second sensor (200), wherein the second sensor (200) comprises an unsealed oscillating membrane (210), wherein the unsealed membrane (210) overlays a cavity (214) in contact with the air outside of the second sensor (200) and wherein the air can flow from the outside of the second sensor (200) to the cavity (214) through at least one opening (212) of the unsealed membrane (210);
- instructions for computing a difference (fd) of the first frequency measurement from the first sensor (250) and the second frequency measurement from the second sensor (200) using a mixer (514) connected to the first oscillator loop (520) and the second oscillator loop (552); and
- instructions for computing a carbon dioxide measurement based on the difference (fd) using a circuit (515) that is coupled to an output of the mixer (514).

**Patentansprüche**

**1.** Vorrichtung, Folgendes aufweisend:

eine erste Oszillatorschleife (520), die einen Drucksensor (250) aufweist,
eine zweite Oszillatorschleife (522), die einen Gassensor (200) aufweist,
einen Mischer (514), der als Eingabe eine erste Frequenzmesswertausgabe (f1) von der ersten Oszillatorschleife (520) und eine zweite Frequenzmesswertausgabe (f2) von der zweiten Oszillatorschleife (522) empfängt und die Differenz des ersten Frequenzmesswerts und des zweiten Frequenzmesswerts ausgibt, und
einen Schaltkreis (515), der an einen Ausgang des Mischers (514) angeschlossen ist,
wobei der Drucksensor (250) einen versiegelten Hohlraum (258) und eine versiegelte Membran (252) aufweist, die durch einen Druckunterschied zwischen einem Gasgemisch außerhalb des Drucksensors (250) und einem Gasgemisch oder Vakuum im versiegelten Hohlraum (258) des Drucksensors (250) abgelenkt wird,
wobei der Gassensor (200) einen Hohlraum (214) und eine unversiegelte schwingende Membran (210) aufweist, die eine Resonanzfrequenz (f2) hat und den in Kontakt mit dem Gasgemisch außerhalb des Gassensors (200) stehenden Hohlraum (214) überdeckt,
wobei das Gasgemisch durch mindestens eine Öffnung (212) der unversiegelten schwingenden Membran (210) von außerhalb des Gassensors (200) zum Hohlraum (214) strömen kann, und
wobei die Vorrichtung dazu ausgelegt ist, eine Molmasse, eine Dichte und/oder eine Viskosität des Gasgemischs in dem von der unversiegelten schwingenden Membran (210) überdeckten Hohlraum (214) zu erfassen.

**2.** Vorrichtung nach Anspruch 1,
wobei der Hohlraum (214) als flacher Hohlraum ausgebildet ist und aufgrund der geringen Höhe des Hohlraums (214) sich das Gasgemisch im Inneren des Hohlraums (214) wie ein gepresster Film verhält, wenn sich eine obere Elektrode (216) des Gassensors (200) zu einer unteren Elektrode (218) des Gassensors (200) bewegt, so dass der Gassensor (200) den Pressfilmeffekt des Gasgemischs nutzt, das in dem Hohlraum (214) eingeschlossen ist.

3. Vorrichtung nach Anspruch 1 oder 2,
   wobei der Gassensor (200) eine obere Elektrode (216) und eine untere Elektrode (218) aufweist und zu einer elektrostatischen, mechanischen Aktivierung der oberen Elektrode (218) ausgelegt ist.

4. Vorrichtung nach einem der Ansprüche 1 bis 3,
   wobei der Gassensor (200) eine Heizvorrichtung nahe am oder in Kontakt mit dem Hohlraum (214) aufweist.

5. Vorrichtung nach Anspruch 4,
   wobei die Temperatur mittels der Heizvorrichtung zyklisch verändert wird.

6. Greifbares und nichtflüchtiges, maschinenlesbares Speichermedium, das mit Instruktionen zur Ausführung durch eine Vorrichtung codiert ist, um Kohlendioxid-($CO_2$)-Gehalte zu messen, wobei das greifbare und nichtflüchtige, maschinenlesbare Speichermedium aufweist:

   - Instruktionen, um eine erste Resonanzfrequenz (f1) einer Luftdruckmessung mittels einer ersten Oszillatorschleife (520) aufzunehmen, die einen ersten Sensor (250) aufweist, wobei der erste Sensor (250) eine versiegelte Membran (252) aufweist und die versiegelte Membran (252) einen versiegelten ersten Hohlraum (258) überdeckt, der ein Gasgemisch oder Vakuum enthält;
   - Instruktionen, um eine zweite Resonanzfrequenz (f2) der Luftmessung mittels einer zweiten Oszillatorschleife (552) aufzunehmen, die einen zweiten Sensor (200) aufweist, wobei der zweite Sensor (200) eine unversiegelte schwingende Membran (210) aufweist, wobei die unversiegelte Membran (210) einen mit der Luft außerhalb des zweiten Sensors (200) in Kontakt stehenden Hohlraum (214) überdeckt, und wobei die Luft von außerhalb des zweiten Sensors (200) durch mindestens eine Öffnung (212) der unversiegelten Membran (210) zum Hohlraum (214) strömen kann;
   - Instruktionen, um eine Differenz (fd) des ersten Frequenzmesswerts vom ersten Sensor (250) und des zweiten Frequenzmesswerts vom zweiten Sensor (100) mittels eines Mischers (514) zu berechnen, der an die erste Oszillatorschleife (520) und die zweite Oszillatorschleife (552) angeschlossen ist; und
   - Instruktionen, um einen Kohlendioxidmesswert auf Grundlage der Differenz (fd) mittels eines Schaltkreises (515) zu berechnen, der an einen Ausgang des Mischers (514) angeschlossen ist.

**Revendications**

1. Dispositif, comprenant
   une première boucle d'oscillateur (520) qui comprend un capteur de pression (250),
   une deuxième boucle d'oscillateur (522) qui comprend un capteur de gaz (200),
   un mélangeur (514) acceptant comme intrant une première sortie de mesure de fréquence (f1) provenant de la première boucle d'oscillateur (520) et une deuxième sortie de mesure de fréquence (f2) provenant de la deuxième boucle d'oscillateur (552), sortant la différence de la première mesure de fréquence et de la deuxième mesure de fréquence, et
   un circuit (515) qui est couplé à une sortie du mélangeur (514),
   sachant que le capteur de pression (250) comprend une cavité (258) hermétique et une membrane (252) hermétique qui est déviée par une différence de pression entre un mélange de gaz à l'extérieur du capteur de pression (250) et un mélange de gaz ou un vide dans la cavité (258) hermétique du capteur de pression (250),
   sachant que le capteur de gaz (200) comprend une cavité (214) et une membrane oscillante (210) non hermétique ayant une fréquence de résonance (f2) et recouvrant la cavité (214) en contact avec le mélange de gaz à l'extérieur du capteur de gaz (200),
   sachant que le mélange de gaz peut s'écouler de l'extérieur du capteur de gaz (200) vers la cavité (214) par au moins une ouverture (212) de la membrane oscillante (210) non hermétique, et
   sachant que le dispositif est configuré pour détecter au moins l'une d'une masse molaire, d'une densité et d'une viscosité du mélange de gaz dans la cavité (214) recouverte par la membrane oscillante (210) non hermétique.

2. Le dispositif de la revendication 1,
   sachant que la cavité (214) est réalisée comme cavité peu profonde et, du fait de la faible hauteur de la cavité (214), le mélange de gaz à l'intérieur de la cavité (214) se comporte comme un film compressé, lorsqu'une électrode supérieure (216) du capteur de gaz (200) se déplace vers une électrode inférieure (218) du capteur de gaz (200) de telle sorte que le capteur de gaz (200) utilise l'effet de film compressé du mélange de gaz qui est piégé dans la cavité (214) .

3. Le dispositif de la revendication 1 ou 2,
sachant que le capteur de gaz (200) comprend une électrode supérieure (216) et une électrode inférieure (218) et est configuré pour un actionnement mécanique électrostatique de l'électrode supérieure (218).

4. Le dispositif de l'une des revendications 1 à 3,
sachant que le capteur de gaz (200) comprend un chauffage proche de ou en contact avec la cavité (214).

5. Le dispositif de la revendication 4,
sachant que la température est cyclée au moyen du chauffage.

6. Support de stockage matériel et non transitoire lisible par machine, codé avec des instructions sur celui-ci pour exécution par un dispositif destiné à mesurer des niveaux de dioxyde de carbone ($CO_2$), sachant que ledit support de stockage matériel et non transitoire lisible par machine comprend :

   - des instructions pour saisir une première fréquence de résonance (f1) de mesure de pression d'air au moyen d'une première boucle d'oscillateur (520) comprenant un premier capteur (250), sachant que le premier capteur (250) comprend une membrane (252) hermétique et la membrane (252) hermétique recouvre une première cavité (258) hermétique comprenant un mélange de gaz ou un vide ;
   - des instructions pour saisir une deuxième fréquence de résonance (f2) de mesure d'air au moyen d'une deuxième boucle d'oscillateur (552) comprenant un deuxième capteur (200), sachant que le deuxième capteur (200) comprend une membrane oscillante (210) non hermétique, sachant que la membrane (210) non hermétique recouvre une cavité (214) en contact avec l'air à l'extérieur du deuxième capteur (200) et sachant que l'air peut s'écouler de l'extérieur du deuxième capteur (200) vers la cavité (214) par au moins une ouverture (212) de la membrane (210) non hermétique ;
   - des instructions pour calculer une différence (fd) de la première mesure de fréquence provenant du premier capteur (250) et de la deuxième mesure de fréquence provenant du deuxième capteur (200) au moyen d'un mélangeur (514) connecté à la première boucle d'oscillateur (520) et à la deuxième boucle d'oscillateur (552) ; et
   - des instructions pour calculer une mesure de dioxyde de carbone sur la base de la différence (fd) au moyen d'un circuit (515) qui est couplé à une sortie du mélangeur (514) .

# FIG 1

| | mbar | cm³ | km | |
|---|---|---|---|---|
| | $-10^{-18}$ | $-10^{-2}$ | $-10^{11}$ | |
| | $-10^{-17}$ | $-10^{-1}$ | $-10^{10}$ | |
| | $-10^{-16}$ | $-1$ | $-10^{9}$ | MOLECULAR FLOW |
| | $-10^{-15}$ | $-10$ | $-10^{8}$ | |
| | $-10^{-14}$ | $-10^{2}$ | $-10^{7}$ | |
| | $-10^{-13}$ | $-10^{3}$ | $-10^{6}$ | |
| | $-10^{-12}$ | $-10^{4}$ | $-10^{5}$ | |
| ULTRA HIGH VACUUM | $-10^{-11}$ | $-10^{5}$ | $-10000$ | |
| | $-10^{-10}$ | $-10^{6}$ | $-1000$ | |
| | $-10^{-9}$ | $-10^{7}$ | $-100$ | |
| | $-10^{-8}$ | $-10^{8}$ | $-10$ | |
| | $-10^{-7}$ | $-10^{9}$ | $-1$ | |
| HIGH VACUUM | $-10^{-6}$ | $-10^{10}$ | $-100$ m | |
| | $-10^{-5}$ | $-10^{11}$ | $-10$ | |
| | $-10^{-4}$ | $-10^{12}$ | $-1$ | |
| | $-10^{-3}$ | $-10^{13}$ | $-10^{-1}$ | |
| MEDIUM VACUUM | $-10^{-2}$ | $-10^{14}$ | $-10^{-2}$ | |
| | $-10^{-1}$ | $-10^{15}$ | $-10^{-3}$ | |
| BACKING VACUUM | $-1$ | $-10^{16}$ | $-10^{-4}$ | LAMINAR FLOW |
| LOW VACUUM | $-10$ | $-10^{17}$ | PATH TRAVELED BY A MOLECULE BEFORE STRIKING ANOTHER | |
| | $-100$ | $-10^{18}$ | | |
| | $-1000$ | $-10^{19}$ | | |

110 PRESSURE (mbar)  MOLECULES IN 1cm³  MEAN FREE PATH

FIG 2A

200

216

TUNGSTEN

THIN BOTTOM
ELECTRODE (Al)

DIELECTRIC
ETCH-STOP
LAYER

W-via's  218  210  214  212

PASSIVATION   W-via's

SHIELDING

ALUMINIUM: M5 CO-Sensor

CMOS back-end

M4   M1, M2, M3

EP 3 191 830 B1

# FIG 2B

TUNGSTEN

THIN BOTTOM
ELECTRODE (Al)

DIELECTRIC
ETCH-STOP
LAYER

250

254 W-via's

256

258

252

PASSIVATION W-via's

SHIELDING

ALUMINIUM: M5 CO-Sensor

CMOS back-end

M4    M1, M2, M3

EP 3 191 830 B1

FIG 2C

200 242

228 229 212 214 224 210 223 218 227 222 221 220 216 226

240 236 233

237 225 230 232 234 235 231 241

FIG 2D

FIG 3

FIG 4

# FIG 5

250

510

f1

520

523
G

200 → 512

f2

522

524
G

514

fd = f1-f2

SH

515

# FIG 6A

## Table 1

| Gas | Volume Fraction | Mass Fraction | Molar Mass | Symbol | Partial Mass | Mol | ppm |
|---|---|---|---|---|---|---|---|
| Oxygen | 20.95 | 23.2 | 32 | $O_2$ | 6.704 | 0.725 | 210048 |
| Nitrogen | 78.09 | 75.47 | 28.02 | $N_2$ | 21.881 | 2.693433262 | 780343 |
| Carbon Dioxide | 0.03 | 0.046 | 44.01 | $CO_2$ | 0.013 | 0.001045217 | 303 |
| Hydrogen | 0.00005 | ~0 | 2.02 | H | 0.000 | | |
| Argon | 0.933 | 1.28 | 39.94 | Ar | 0.373 | 0.032048072 | 9285 |
| Neon | 0.0018 | 0.0012 | 20.18 | Ne | 0.000 | 5.94648E-05 | 17 |
| Helium | 0.0005 | 0.00007 | 4 | He | 0.000 | 0.0000175 | 5 |
| Krypton | 0.0001 | 0.0003 | 83.8 | Kr | 0.000 | 3.57995E-06 | 1 |
| Xenon | $9(10^{-6})$ | 0.00004 | 131.29 | Xe | | 3.04669E-07 | 0 |
| | | | | **SUM:** | **28.971** | | **1000002** |

# FIG 6B

## Table 2

| Gas | Partial Mass | Mol | ppm | increased $CO_2$ | $CO_2$ DELTA | Partial Mass |
|---|---|---|---|---|---|---|
| Oxygen | 6.704 | 0.725 | 210048 | 210006 | | 6.72 |
| Nitrogen | 21.881 | 2.693433262 | 780343 | 780190 | | 21.861 |
| Carbon Dioxide | 0.013 | 0.001045217 | 303 | 500 | 197 | 0.022 |
| Hydrogen | 0.000 | | | | | 0 |
| Argon | 0.373 | 0.032048072 | 9285 | 9283 | | 0.371 |
| Neon | 0.000 | 5.94648E-05 | 17 | 17 | | 0 |
| Helium | 0.000 | 0.0000175 | 5 | 5 | | 0 |
| Krypton | 0.000 | 3.57995E-06 | 1 | 1 | | 0 |
| Xenon | | 3.04669E-07 | 0 | 0 | | |
| SUM: | 28.971 | | 1000002 | 1000002 | | 28.974 |

# FIG 6C

## Table 3

| Kelvin | Celsius | mbar | RH% | ppm |
|---|---|---|---|---|
| 298.15 | 25 | 1013.25 | 50 | 15619 |
| 298.15 | 25 | 1013.25 | 60 | 18743 |
| 298.15 | 25 | 1013.25 | 70 | 21867 |
| 298.15 | 25 | 1013.25 | 80 | 24990 |

# FIG 6D

## Table 4

| Kelvin | Celsius | mbar | RH | ppm |
|---|---|---|---|---|
| 298.15 | 25 | 1013.25 | 50 | 15619 |
| 323.15 | 50 | 1013.25 | 50 | 60861 |
| 348.15 | 75 | 1013.25 | 50 | 190180 |
| 373.15 | 100 | 1013.25 | 50 | 499820 |

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2009071746 A1
- EP 2700928 A2
- US 20020115198 A1

**Non-patent literature cited in the description**

- **MATTHIJS SUIJLEN.** Model-based design of MEMS resonant pressure sensors. *NXP 2011, Thesis,* 14 August 2014, http://alexan-dria.tue.nl/extra2/716458.pdf